# EUROPÄISCHE PATENTANMELDUNG

(11) **EP 0 943 605 A2**
(43) Veröffentlichungstag der Anmeldung: **22.09.1999**
(21) Anmeldenummer: 99104840.6
(22) Anmeldetag: 11.03.1999
(51) Int. Cl.: C07C 253/00, C07C 255/04

(54) **Verfahren zur Herstellung von Malonsäuredinitril**

(30) Priorität: 19.03.1998 CH 66598
(71) Anmelder: Lonza AG, 4052 Basel (CH)
(72) Erfinder: Chen, Peter, Prof. Dr., 8044 Zürich (CH); Hoffner, Johannes, Dr., 8053 Zürich (CH); Müller, André, 8954 Geroldswil (CH); Fuchs, Rudolf, Dr., 1950 Sion (CH)

(57) **Zusammenfassung**

Es wird ein neues Verfahren zur Herstellung von Malonsäuredinitril beschrieben, worin ein Isonitril gegebenenfalls in Gegenwart eines Nitrils einer Hochtemperaturbehandlung unterzogen wird.

## Beschreibung

Die Erfindung betrifft ein neues Verfahren zur Herstellung von Malonsäuredinitril oder von Derivaten des Malonsäuredinitrils der allgemeinen Formel 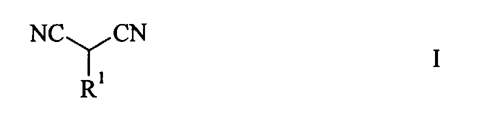 worin R¹ für Wasserstoff, eine Alkylgruppe oder eine Cycloalkylgruppe steht

Malonsäuredinitril ist ein Ausgangs- und Zwischenprodukt von zentraler Bedeutung für die Herstellung von einer ausserordentlichen Vielfalt von z. B. pharmazeutischen oder agrochemischen Wirkstoffen (Ullmann's Encyklopädie der technischen Chemie, 4. neub. und erw. Auflage , Verlag Chemie Weinheim, Band 16, S. 419-423).

Zur Herstellung von Malonsäuredinitril sind zahlreiche Verfahren bekannt, grosstechnische Bedeutung hat allerdings lediglich die Hochtemperaturumsetzung von Acetonitril mit Chlorcyan bei Temperaturen von über 700 °C erlangt.

Die Aufgabe der Erfindung bestand darin, alternative Verfahren mit Potential für den grosstechnischen Einsatz zu entwickeln.
Die Aufgabe konnte gelöst werden mit dem neuen Verfahren gemäss Patentanspruch 1.

Erfindungsgemäss wird ein Isonitril der allgemeinen Formel

R² ― NC II

worin R² für eine Alkylgruppe oder eine Cycloalkylgruppe steht, gegebenenfalls in Gegenwart von einem Nitril der allgemeinen Formel

R³ ― CN III

worin R³ für Wasserstoff, eine Alkylgruppe oder eine Cycloalkylgruppe steht, bei einer Temperatur von 700 °C bis 1000 °C umgesetzt.

Unter einer Alkylgruppe wird zweckmässig eine C₁₋₆-Alkylgruppe, namentlich Methyl, Ethyl, n-Propyl, i-Propyl, n-Butyl, i-Butyl, t-Butyl, Pentyl und seine Isomeren oder Hexyl und seine Isomeren verstanden. Bevorzugte Alkylgruppe ist Methyl.
Cycloalkyl steht zweckmässig für C₃₋₆-Cycloalkyl, namentlich Cyclopropyl, Cyclobutyl, Cyclopentyl oder Cyclohexyl.
Bevorzugt ist die Umsetzung von Methylisonitril mit Acetonitril.

Da die Isonitrile der allgemeinen Formel II zu den entsprechenden Nitrilen isomerisieren können, ist es möglich, bei deren Umsetzung mit den Nitrilen der allgemeinen Formel III Derivate des Malonsäurenitrils zu erhalten, deren Gruppe R¹ entweder der Gruppe R² vom Isonitril oder der Gruppe R³ vom Nitril entsprechen kann. Dies natürlich unter der Voraussetzung, dass R² und R³ verschieden sind. Entsprechend kann eine solche Umsetzung beliebige Mischungen der Derivate des Malonsäurenitrils liefern.

Die Isonitrile der allgemeinen Formel II sind entweder kommerziell erhältlich oder können nach bekannten Literaturverfahren z. B. Angew. Chemie, 1965, 77, 492-504 oder J. Am. Chem. Soc., 1981, 103, 767-772 synthetisiert werden.

Die erfindungsgemässe Hochtemperaturumsetzung verläuft vorzugsweise bei einer Temperatur von 800 °C bis 950 °C.
Üblicherweise wird die Reaktion in einem Rohrreaktor, der gegebenenfalls mit geeigneten Füllkörpern versehen ist, durchgeführt.
Die Umsetzungszeit beträgt in der Regel wenige Sekunden.

Das Malonsäuredinitril kann aus dem Reaktionsprodukt z.B. durch Extraktion mit einem geeigneten Lösungsmittel isoliert werden.

### Beispiel 1:

### Synthese und Reinigung von Malonsäuredinitril durch Thermolyse von Methylisocyanid und Acetonitril

2 ml Methylisocyanid wurden mit 3 ml Acetonitril verdünnt und mittels einer Spritze in einen Dampfstrom einer Acetonitrildestillation (15 g während 20 Minuten) eingetragen. Der Dampfstrom wurde in ein auf 870 °C erwärmtes Quarz-Pyrolyserohr (Länge 30 cm, Innendurchmesser 2,5 cm) eingetragen. Das Reaktonsprodukt wurde in einer auf-50 °C gekühlten Kühlfalle aufgefangen.
Der Kühlfallen-Rückstand wurde am Rotavapor eingeengt und der Gehalt der Reaktionsprodukte (516 mg) wurde durch ¹H-NMR bestimmt.
- Malonsäuredinitril: 18%
- Succinonitril: 1%
- Fumaronitril und Maleinnitril: 1%

Das Rohprodukt wurde mit Chloroform extrahiert. In diesem Extrakt waren nur Malonsäuredinitril und Succinonitril löslich.

### Beispiel 2:

### Synthese von Malonsäuredinitril durch Thermolyse von Methylisocyanid und Acetonitril

2 ml Methylisocyanid wurden mit 3 ml Acetonitril verdünnt und mittels einer Spritze in einen Dampfstrom einer Acetonitrildestillation (15 g während 20 Minuten) eingetragen. Der Dampfstrom wurde in ein auf 920 °C erwärmtes, equlibriertes Quarz-Pyrolyserohr (Länge 30 cm, Innendurchmesser 2,5 cm) eingetragen. Das Reaktionsprodukt wurde in einer auf -50 °C gekühlten Kühlfalle aufgefangen.
Der Kühlfallen-Rückstand wurde am Rotavapor eingeengt und der Gehalt der Reaktionsprodukte (1400 mg) wurde durch ¹H-NMR bestimmt.
- Malonsäuredinitril: 40%
- Succinonitril: 1,5%
- Fumaronitril und Maleinnitril: 9%

Analog zu Beispiel 2, aber bei unterschiedlichen Temperaturen des Quarzrohres, wurden die nachfolgenden Beispiele durchgeführt.

| | Temperatur in °C | Acrylonitril | Succinonirril | Fumaro- und Maleinnitril | Malonsäuredinitril |
|---|---|---|---|---|---|
| 3 (Vergl.) | 670 | | 0 | 0 | 0 |
| 4 (Erf.) | 770 | | 1 | 0,2 | 1,1 |
| 5 (Erf.) | 820 | | 0,6 | 0,4 | 4 |
| 6 (Erf.) | 845 | | 0,8 | 1 | 12 |
| 7 (Erf.) | 870 | | 0,5 | 2,5 | 18 |
| 8 (Erf.) | 895 | 2 | 1 | 5 | 33 |
| 9 (Erf.) | 920 | 10 | 1,5 | 8 | 40 |
| 10 (Erf.) | 945 | 33 | 1 | 17 | 35 |
| 11 (Erf.) | 970 | 28 | 2 | 20 | 29 |
| 12 (Vergl.) | 1070 | 0 | 0 | 0 | 0 |

## Patentansprüche

1. Verfahren zur Herstellung von Malonsäuredinitril oder von Derivaten des Malonsäuredinitrils der allgemeinen Formel 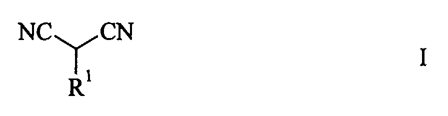 worin R¹ für Wasserstoff, eine Alkylgruppe oder eine Cycloalkylgruppe steht, dadurch gekennzeichnet, dass ein Isonitril der allgemeinen Formel
R² ― NC II
worin R² für eine Alkylgruppe oder eine Cycloalkylgruppe steht, gegebenenfalls in Gegenwart von einem Nitril der allgemeinen Formel
R³ ― CN III
worin R³ für Wasserstoff, eine Alkylgruppe oder eine Cycloalkylgruppe steht, bei einer Temperatur von 700 °C bis 1000 °C umgesetzt wird.

2. Verfahren nach Patentanspruch 1, dadurch gekennzeichnet, dass als Isonitril der allgemeinen Formel II das Methylisonitril und als Nitril der allgemeinen Formel III das Acetonitril gewählt wird.

3. Verfahren nach einem der Patentansprüche 1 oder 3, dadurch gekennzeichnet, dass die Umsetzungstemperatur 800 °C bis 950 °C beträgt.
